# EUROPEAN PATENT APPLICATION

(11) **EP 1 230 936 A1**
(43) Date of publication of application: **14.08.2002**
(21) Application number: 02075479.2
(22) Date of filing: 06.02.2002
(51) Int. Cl.: A61L 2/28

(54) **Method and device for determining the process conditions in sterilization**

(30) Priority: 07.02.2001 NL 1017308
(71) Applicant: Answers, Solutions and Know-how B.V. i.o., 3821 AJ Amersfoort (NL)
(72) Inventor: van Doornmalen, Josephus P. C. M., 5688 AW Oirschot (NL); Verschueren, Michael, 5631 CH Eindhoven (NL)
(74) Representative: Prins, Adrianus Willem

(57) **Abstract**

The invention relates to a method for determining the sterilization conditions for surface sterilization adjacent a reference surface which is in communication with a sterilization space. The sterilant has only limited access to the reference surface. The method comprises the step of generating an electronic signal which corresponds to the sterilization conditions adjacent the reference surface. The method can comprise the further step of generating a reference signal; and determining the deviation of the electronic signal with respect to the reference signal. The reference signal can correspond to the degree of saturation of the sterilant in the sterilization space.

## Description

The invention relates to a method for determining the sterilization conditions for surface sterilization with a sterilant adjacent a reference surface which is in communication with a sterilization space, the sterilant having only limited access to the reference surface.

Sterilization media kill microorganisms that are present on surfaces of objects to be sterilized, such as surgical instruments. Such surfaces are understood to include: microsurfaces such as porous structures. The physical parameters that are of importance for a sterilization process comprise in particular the degree of saturation of the sterilant, and the pressure and temperature, which prevail in a sterilization space as a function of time. In the application, these parameters are designated by the term "sterilization conditions".

To achieve adequate sterilization, the norm used is that the chance of finding a microorganism on a surface of an object to be sterilized should be less than 1:1,000,000. The assumption here is that it is necessary, for obtaining a sufficient sterilization, that steam be 100% saturated. In fact, in that case, upon condensation of the steam as a result of released phase heat (also called latent heat) an optimum heat transfer takes place from the steam to the surface to be sterilized. If saturation is not complete, microorganisms can survive, despite an executed sterilization program, and sterility is guaranteed insufficiently.

In practice, to realize the proper process conditions in sterilization, use is made of standard programs. These involve, for instance, sterilization with steam in an autoclave for 3 minutes at 134 °C at a pressure of about 305 kPa. Other standard programs comprise, for instance, 20 minutes of sterilization at a temperature of 120 °C, or 15 minutes at 121 °C. In these programs, it is always assumed that saturation is 100%.

To arrive at saturated steam, the air present needs to be removed, and the steam must penetrate to all surfaces present in the sterilization space. Conventionally, the air present is displaced by steam, by alternately creating a partial vacuum in the sterilization space and subsequently introducing saturated steam into the sterilization space. By increasing the number of 'vacuum pulses', penetration is improved and the steam also penetrates to surfaces that are very difficult to access. Such a process in which several times a vacuum is applied and the pressure is increased with steam is called a fractionated prevacuum process. These processes are convection-controlled and often take less time than a diffusion-controlled process.

To determine if upon an executed sterilization process the required process conditions have in effect been achieved, it is known to introduce a so-called process challenge device into a sterilization space. Such a device is described, for instance, in the European patent application EP-A-0,416,504. An alternative to this known test (also known as the Bowie & Dick test) has been laid down in a new European norm (preliminary European Norm prEN 13060). In this norm, a so-called test helix is proposed, consisting of a helically configured teflon tube of about 1.5 m, having an outside diameter of 3 mm and an inside diameter of 2 mm. The helix is open at one end. The other end terminates in a chamber in which an indicator can be arranged. Prior to a sterilization process, in the chamber an indicator is arranged and the helix is arranged in a sterilization space. Contained in the indicator is, for instance, a steam-sensitive ink. The ink, upon a particular extent of exposure to steam, exhibits a complete color change. The idea behind the test instrument applied is that the sterilant has only limited access to the reference surface in that the steam supplied to the sterilization space can reach the surface of the indicator least easily. The other surfaces of objects present in the sterilization space are therefore supposed to be sterile if upon completion of the process the indicator indicates a positive result. To that end, the indicator is removed from the chamber and interpreted. The above conventional methods have as a drawback that a sterilization is assessed on the basis of a qualitative assessment. The known method may therefore in some cases be insufficiently accurate.

The object of the invention is to provide a method and device enabling a more accurate measurement to be performed.

This object is achieved by a method according to the type described in the preamble, which method comprises the step of generating an electronic signal from which the sterilization conditions adjacent the reference surface can be derived. The reference surface can be arranged in a reference space. Consequently, on the basis of a quantitative measurement, it can be determined whether the norm for predefined sterilization conditions has been met.

The method according to the invention has as an advantage that the result of the measurement is much more accurate than in the conventional method. What is thereby avoided is that, as is the case in the conventional methods, a test is ambiguous, for instance in that it shows a partial color change. Also, the circumstances under which a sterilization is taking place can be analyzed better than in the conventional method. Additionally, with the aid of the method according to the invention, the process conditions can be adjusted, so that for all surfaces present in the sterilization space, the norm for the correct sterilization conditions is satisfied.

In a preferred embodiment, the method can comprise the further step of generating a reference signal and determining the deviation of the electronic signal from the reference signal. Although a fixed reference value can be taken as a starting point, which value corresponds, for instance, with predetermined process conditions, a further preferred embodiment relates to a method in which the reference signal corresponds to sterilization conditions measured in the sterilization space. If, for instance, the measured signal exhibits a predefined deviation from the reference signal, the sterilization can be rejected because this is an indication that the norm for the correct sterilization conditions is not being met for all surfaces present in the sterilization space.

Also, the process conditions can be accurately registered, on the basis of which the sterilization process, in the event of a predetermined deviation of the signal, can be adjusted. Adjusting the sterilization process can comprise prolonging the duration of sterilization, varying the pressure of the sterilant or varying the sterilization temperature.

The method can comprise the step of determining the condensation on the reference surface. In a preferred embodiment, the method comprises thermally insulating the reference surface with respect to the sterilization space; measuring a temperature adjacent the reference surface; measuring a temperature in the sterilization space; determining the difference in the measured temperatures; and deriving from the difference measurement of these temperatures the condensation on the reference surface.

The amount of condensation is indicative of the presence of saturated steam. In fact, through the capacitive action of the wall of the lumen, the temperature in the lumen will lag behind the temperature in the sterilization chamber. As a consequence, on the inner wall condensation will occur, provided that the degree of saturation adjacent the reference surface is high enough. Upon a pressure fall, the course of the temperature, as a result of evaporation, will deviate from the course of the temperature in a situation without condensation.

Preferably, the method comprises the further step of measuring the pressure in the sterilization space; and, with the aid of an electronic computing unit, deriving the degree of saturation in the reference space from the temperature and pressure relations for the sterilant.

By quantifying the deviant temperature curve on the basis of the thermal properties of the material and relating pressure (p) and measured temperature (T) in the sterilization space and the reference space, the degree of saturation can be determined.

The method can further comprise the step of measuring the pressure adjacent the reference surface. This is of importance in particular if the sterilant has such limited access to the reference surface that the pressure can lag behind the pressure prevailing in the sterilization space, for instance if the reference surface is situated at the end of a very narrow lumen. In that case, the degree of saturation adjacent the reference surface is also influenced by the pressure which is measured in situ.

The invention further relates to a device for determining sterilization conditions, comprising a reference chamber which can be placed in a sterilization space, which chamber is provided with a transducer, with the aid of which the degree of saturation of the sterilant adjacent the transducer can be determined and which chamber is provided with an opening having a geometry such that a sterilant, with respect to the sterilization space, has relatively difficult access to the transducer. According to the invention, the device is characterized in that the transducer can produce an electronic signal from which the sterilization conditions adjacent the transducer can be derived.

Limited access is understood to mean that owing to the geometry of the chamber the sterilant can relatively less easily penetrate to the reference surface, for instance in that the reference surface is disposed at the end of a narrow lumen or behind a porous or (semi)permeable structure.

In a preferred embodiment, the transducer comprises an electronic temperature sensor, and the chamber is provided with at least partially thermally insulated walls. The device can comprise a temperature sensor for measuring the temperature in the sterilization space, a pressure sensor for measuring the pressure in the sterilization space and an electronic computing unit, for deriving the sterilization conditions adjacent the transducer from the measured temperature and pressure. With a device according to this preferred embodiment, the deviation of the temperature curve, as set out above, can be determined and the degree of saturation of the sterilant can be derived. It is then preferred that the temperature sensor has a relatively low heat capacity, in particular low with respect to the reference surface.

The device can comprise an electronic pressure sensor, for measuring the pressure adjacent the transducer.

The chamber can have a geometry of a hollow lumen. The chamber can be coupled to a hollow body, which has a characteristic geometry for objects to be sterilized. The body can comprise a hollow lumen.

The invention will be further elucidated with reference to the drawing. In the drawing:
Fig. 1 is a schematic representation of a first embodiment of the device according to the invention; and
Fig. 2 is a schematic representation of a second embodiment of the device according to the invention.

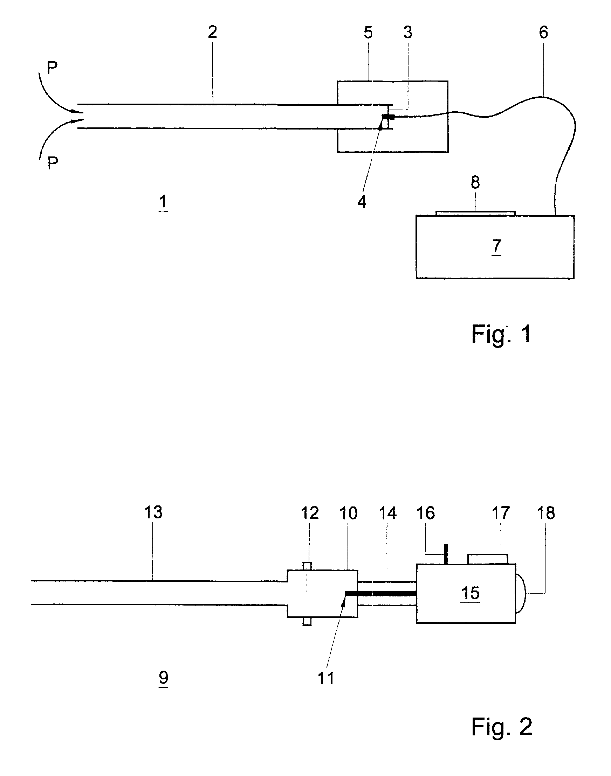

Fig. 1 represents a device 1 for determining the sterilization conditions in a sterilization space. The device 1 comprises a reference chamber in the form of a narrow lumen 2. The lumen 2 can be placed in a sterilization space (not shown), in which a sterilant, such as steam, is introduced. The lumen 2 is closed at one end; the terminal inner wall 3 is provided with a transducer 4, with the aid of which the temperature and the degree of saturation of the sterilant adjacent the transducer can be determined. The geometry of the lumen is such that a sterilant, with respect to the other parts of the sterilization space, has relatively difficult access to the transducer. This access is schematically illustrated with arrows P. Although other configurations may also be suitable, the lumen 2 can have, for instance, the dimensions mentioned in the introduction: a tube of about 1.5 m, having an outside diameter of 3 mm and an inside diameter of 2 mm. The transducer represented in Fig. 1 comprises a temperature sensor 4, which can produce an electronic signal with the aid of which the sterilization conditions adjacent the wall 3 can be determined. Further, the lumen 2 is provided with partially thermally insulated walls 5 adjacent the closed end 3. The signals coming from the sensor 4 are coupled via a signal line 6 to a processing unit 7. The unit 7 is also in communication with a pressure sensor 8, which can measure the pressure in the sterilization space. The processing unit 7, by relating the measured temperature to the measured pressure, can determine the degree of saturation of the sterilant adjacent the temperature sensor.

The processing unit 7 can store the performed measurements in a database on which further analysis can be performed. Although as so-called challenge device, i.e., as assessment instrument for assessing the conditions in a sterilizer, the device should function independently of a sterilizer, it is possible, in another embodiment, that processing unit 7 constitutes a feedback for controlling the sterilizer, whereby factors such as duration of sterilization, sterilant supply and sterilization temperature can be varied.

The embodiment represented in Fig. 2 can be placed as one whole in a sterilization space, and function as a test appliance 9, with the aid of which the process conditions in a sterilization can be assessed. To that end, the appliance 9 comprises a chamber 10, in which an electronic temperature sensor 11 is arranged. The chamber comprises a coupling element 12, for instance in the form of a screw coupling, so that it can be connected with bodies having a geometry corresponding to objects to be sterilized. In Fig. 2 such a body is represented in the form of a hollow lumen 13. The temperature sensor is thermally insulated with respect to the sterilization space by means of thermally insulating walls 14, and connected with an electronic processing unit 15. The unit is heat-resistant, and comprises a further temperature sensor 16, for measuring the temperature in the sterilization space, and a pressure sensor 17, for measuring the pressure in the sterilization space. The processing unit relates the temperature measured by sensor 11 to the pressure measured by sensor 17 and to the temperature measured by sensor 16 in the sterilization space, and derives therefrom the degree of saturation of the sterilant in the chamber 10. Also, the degree of saturation of the sterilant in the sterilization space is determined, by relating the temperature measured by sensor 16 to the measured pressure. In the event of a predetermined deviation of the sterilization conditions determined in the chamber, with respect to the conditions determined in the sterilization space, an indicator lamp 18, for instance a LED, can start to blink red, to indicate that a sterilization is not proceeding properly or has not proceeded properly. If such a deviation does not occur, the LED 18 can show a green signal light, indicating that a sterilization is correctly performed.

It will be clear to one skilled in the art that the invention is not limited to the exemplary embodiments described with reference to the drawings, but can encompass all kinds of variations thereon. Thus, in Fig. 1, the processing unit 7 and the pressure sensor 8 are represented as being integral, but other embodiments are conceivable as well, whereby, for instance, a pressure sensor is arranged in a wall of a sterilizer, and/or the signals of pressure sensor 8 and temperature sensor 4 are passed to a processing unit present outside a sterilization space. Physical parameters of importance can also be derived in an indirect manner. Although in the present application reference has chiefly been made to steam sterilization, it is also possible, in the invention, to use other sterilization media, such as gas sterilization or plasma sterilization. Such variations are understood to fall within the scope of invention of the following claims.

## Claims

1. A method for determining the sterilization conditions for surface sterilization with a sterilant adjacent a reference surface which is in communication with a sterilization space, the sterilant having only limited access to the reference surface, **characterized in that** the method comprises the step of generating an electronic signal from which the sterilization conditions adjacent the reference surface can be derived.

2. A method according to claim 1, **characterized in that** the reference surface is arranged in a reference space.

3. A method according to claim 1, **characterized in that** the method comprises the further step of generating a reference signal and determining the deviation of the electronic signal with respect to the reference signal.

4. A method according to claim 3, **characterized in that** the reference signal corresponds to sterilization conditions measured in the sterilization space.

5. A method according to at least one of claims 3 or 4, **characterized in that** in the event of a predetermined deviation of the signal, the sterilization process is adjusted.

6. A method according to claim 5, **characterized in that** adjusting the sterilization process comprises prolonging the duration of sterilization.

7. A method according to at least one of claims 5 or 6, **characterized in that** adjusting the sterilization process comprises supplying sterilant.

8. A method according to at least one of claims 5-8, **characterized in that** adjusting the sterilization conditions comprises varying the sterilization temperature.

9. A method according to at least one of the preceding claims,
**characterized in that** the method comprises the step of rejecting the sterilization if the signal has a predetermined deviation with respect to the reference signal.

10. A method according to at least one of the preceding claims,
**characterized in that** the method comprises the step of determining condensation on the reference surface.

11. A method according to claim 10, **characterized in that** the method comprises thermally insulating the reference surface with respect to the sterilization space; measuring a temperature adjacent the reference surface; measuring a temperature in the sterilization space; determining the difference in the measured temperatures; and deriving, from the difference measurement of these temperatures, condensation present on the reference surface.

12. A method according to claim 11, **characterized in that** the method comprises the further step of measuring the pressure in the sterilization space; and, with the aid of an electronic computing unit, deriving the degree of saturation in the reference space from the temperature and pressure relations for the sterilant.

13. A method according to claim 12, **characterized in that** the method comprises the further step of measuring the pressure adjacent the reference surface.

14. A device for determining sterilization conditions, comprising a reference chamber which can be placed in a sterilization space, which chamber is provided with a transducer, with the aid of which the degree of saturation of the sterilant adjacent the transducer can be determined and which chamber is provided with an opening having a geometry such that a sterilant, with respect to the sterilization space, has relatively difficult access to the transducer, **characterized in that** the transducer can produce an electronic signal from which the sterilization conditions adjacent the transducer can be derived.

15. A device according to claim 14, **characterized in that** the transducer comprises an electronic temperature sensor and that the chamber is provided with at least partially thermally insulated walls.

16. A device according to claim 15, **characterized in that** the device further comprises a temperature sensor for measuring the temperature in the sterilization space, a pressure sensor for measuring the pressure in the sterilization space and an electronic computing unit, for deriving the sterilization conditions adjacent the transducer from the measured temperature and pressure.

17. A device according to claim 15 or 16, **characterized in that** the temperature sensor has a relatively low heat capacity with respect to the reference surface.

18. A device according to claims 15-17, **characterized in that** the device comprises an electronic pressure sensor, for measuring the pressure adjacent the transducer.

19. A device according to at least one of the claims 14-18, **characterized in that** the chamber has the geometry of a hollow lumen.

20. A device according to at least one of the claims 14-19, **characterized in that** the chamber comprises a coupling element, with the aid of which the chamber can be coupled to a hollow body, which body has a characteristic geometry for objects to be sterilized.

21. A device according to claim 20, **characterized in that** the body comprises a hollow lumen.
